Europäisches Patentamt

(19) European Patent Office

Office européen des brevets

(11) Publication number : **0 658 621 A1**

(12) **EUROPEAN PATENT APPLICATION**

(21) Application number : 94309336.9

(22) Date of filing : 14.12.94

(51) Int. Cl.⁶ : **C12N 1/08**, C12N 1/06, C12Q 1/68

(30) Priority : **14.12.93 US 166773**

(43) Date of publication of application :
**21.06.95 Bulletin 95/25**

(84) Designated Contracting States :
**DE FR GB IT**

(71) Applicant : **Becton Dickinson and Company**
**One Becton Drive**
**Franklin Lakes, New Jersey 07417-1880 (US)**

(72) Inventor : **Keating, William**
**500 Woodcroft Parkway, 18-A**
**Durham, NC 27713 (US)**
Inventor : **Walker, Terrance G.**
**209 Mt. Bolus Road, Chapel Hill**
**NC 27709 (US)**

(74) Representative : **Ruffles, Graham Keith**
**MARKS & CLERK,**
**57-60 Lincoln's Inn Fields**
**London WC2A 3LS (GB)**

(54) **Enzymatic removal of non-cibles nucleic acid from biological samples.**

(57)    The present invention relates to a method for removal of contaminating nucleic acid from a biological sample by exposing the sample to a unique combination of enzymes. The combination of enzymes includes an enzyme which cleaves nucleic acid in an endonuclitic manner and an enzyme which cleaves nucleic acid in an exonuclitic manner. After exposure of the sample to the combination of enzymes, any nucleic acid to be detected may be amplified prior to detection.

EP 0 658 621 A1

## BACKGROUND OF THE INVENTION

Preparation of nucleic acid fragments for amplification may involve numerous steps including collection of an appropriate biological sample, liquification of the sample, lysis of the cells in the sample, dissociation of the DNA by phenol/chloroform extraction, and binding-elution of DNA to glass surfaces. However, amplification of the desired nucleic acid fragments is often hampered by the presence of other non-target nucleic acid fragments which interfere with the amplification process by hybridizing to primers and causing amplification of undesired products, overwhelming target specific amplification. The enzymes (polymerases) are often exhausted from extension of non-target nucleic acid.

Conventional techniques for addressing the problems created by non-target nucleic acid fragments are time consuming, tedious, and/or labor intensive. Some of these methods involve the culturing of the biological sample to increase the number of organisms which would contain the desired nucleic acid fragment prior to amplification (see Brisson-Noel et al., The Lancet, Nov. 4, 1989, pp. 1069-1071; and Pierre et al., J. Clin. Microbiol., vol. 29, April 1991, pp. 712-717). However, these methods add extra days to the process. Others skilled in the art only analyze purified cultures rather than clinical samples (see Patel et al., J. Clin. Microbiol, vol. 28, March 1990, pp. 513-518; Hance et al., Molecular Microbiol., vol. 3, 1989, pp. 843-849; and Fries et al., J. Clin. Microbiol., vol. 29, August 1991 pp. 1744-1747). Still others have used tedious DNA extraction methods to purify DNA prior to amplification (see DeWit et al., J. Clin. Microbiol., vol. 28, Nov. 1990, pp 2437-2441; and Sjobring et al., J. Clin Microbiol., vol. 28, October 1990, pp. 2200-2204).

## SUMMARY OF THE INVENTION

The present invention overcomes the time consuming, tedious and labor intensive characteristics of the conventional techniques as it relates to a method for removal of non-target nucleic acid from a biological sample which may contain a microorganism with nucleic acid to be detected, the method comprising exposing the biological sample to a combination of enzymes which cleave double stranded or single stranded nucleic acid in an endonuclitic and exonuclitic manner.

A particularly useful combination of enzymes is (a) DNase I; (b) S 1 nuclease, and (c) Mung Bean Nuclease. The removal of the non-target nucleic acid from the biological sample is generally more effective when the sample is liquified prior to exposure to the combination of enzymes. After exposure to the combination of enzymes, the microorganism generally is lysed to release nucleic acid which is then amplified prior to detection.

## BRIEF DESCRIPTION OF THE FIGURES

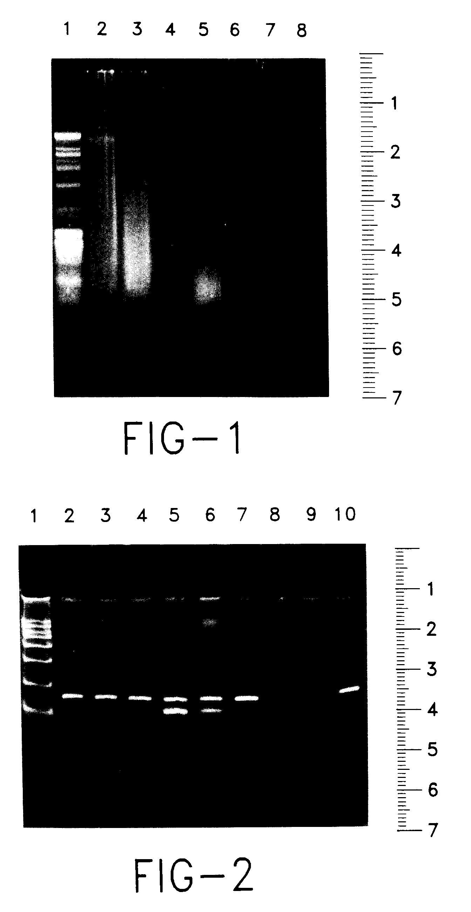

The various objects advantages and novel features of the invention will be more readily appreciated from the following detailed description when read in conjunction with the appended figures in which Figures 1-9 show various electrophoretic gels which evidence the effects of various combinations of enzymes as more fully described in Examples 1-7.

## DETAILED DESCRIPTION OF THE INVENTION

The present invention relates to a method for removal of non-target nucleic acid from a biological sample which may contain a microorganism with nucleic acid to be detected (the target nucleic acid). The removal of the non-target nucleic acid is accomplished at least in part by exposing the biological sample to a combination of enzymes which cleave nucleic acids in different ways.

The combination of enzymes includes at least one enzyme which cleaves nucleic acid in an endonuclitic manner and at least one enzyme which cleaves nucleic acid in an exonuclitic manner. The endonuclitic enzyme cleaves double stranded and/or single stranded nucleic acid or nucleic acid fragments at locations interior, or between the 5' and 3' ends of the nucleic acid or fragment. In contrast, the exonuclitic enzyme cleaves double stranded and/or single stranded nucleic acid or fragment at the 5' and/or 3' ends of the nucleic acid or fragment.

One example of a particularly effective combination of enzymes includes: (1) an enzyme which cleaves double stranded nucleic acid; (2) an enzyme which cleaves the 3' ends of nucleic acid; and (3) an enzyme which cleaves 5' ends of single stranded nucleic acid. The enzyme which cleaves 3' ends of nucleic acid may be effective with single stranded or double stranded nucleic acid. When the nucleic acid is double stranded, the third enzyme will be cleaving the 5' ends of resultant single stranded overhangs.

Alternatively, the combination of enzymes could include: (1) an enzyme which cleaves double stranded nucleic acid; (2) an enzyme which cleaves the 5' ends of nucleic acid; and (3) an enzyme which cleaves 3' ends of single stranded nucleic acid. With this combination, if the nucleic acid is double stranded, the third enzyme will be cleaving 3' ends of resultant single stranded overhangs. Provided with the table of enzymes and their actions below and the descriptions and Examples herein, one skilled in the art could select effective combinations of enzymes which cleave in endonuclitic and exonuclitic manners without having to engage in undue experimentation.

The amounts of the enzymes in the combination are dependent on the amount of the biological sample. Generally, however, the amounts of the enzymes are quantified as units as defined by the commercial vendor of the enzyme. For example, a DNase I unit is defined as follows: one (1) unit causes an increase in absorbance at 260 nm of .001 per minute per milliliter when acting upon highly polymerized DNA at 25°C and pH 5.0 under specified conditions of Kunitz, M. (1950) J. Gen. Physiol. vol 33, p349.

Generally, when only an endonuclitic and exonuclitic enzyme are used, the units ratio may range between about 10:1 and about 1:10, but is preferably about 1:1. However, the preferred units ratio of the combination of (a) enzyme which cleaves double stranded nucleic acid, (b) enzyme which cleaves 3' ends of nucleic acid, and (c) enzyme which cleaves 5' ends of single stranded nucleic acid is about 2:1:1. Typically, the biological sample to be processed contains from 0 to 500 micrograms of DNA, and the three enzymes in the combination are present in amounts from about 10 units: 5 units: 5 units to about 500 units: 250 units: 250 units, but preferably about 50 units:25 units: 25 units .

Suitable exemplary endonuclitic and exonuclitic enzymes for use in the present invention and their respective vendors or suppliers and mechanisms of action are set forth in the table below.

Table 1

Endonuclitic and Exonuclitic Enzymes

| Enzyme | Vendor | Mechanism of Action |
|---|---|---|
| DNase I | Boehringer Mannheim | Cleaves double stranded nucleic acid by splitting phosphodiester linkages at optimally pH 7.8 under magnesium or calcium at 0° - 90°C (optimally 37°C) |
| Exonuclease III | Boehringer Mannheim | Cleaves 3' ends of double stranded nucleic acid by hydrolysis of phosphodiester bonds and stepwise removal of 5' mononucleotides from 3' hydroxyl termini of double stranded nucleic acid (optimal temperature is 37°C in 66 mM TRIS-HCl pH 8.0 and 6 mM $MgCl_2$ |
| Endonuclease VII | Boehringer Mannheim Life Technologies, Inc. | Degrades both 3' and 5' ends of single stranded nucleic acid. |

| | | |
|---|---|---|
| Micrococcal nuclease | Boehringer Mannheim | Degrades both 3' and 5' ends of single stranded nucleic acid. |
| Exonuclease I | Boehringer Mannheim United States Biochemical | Degrades both 3' and 5' ends of single stranded nucleic acid. |
| Mung Bean Nuclease | New England Biolabs | Cleaves 5' ends of single stranded nucleic acid by stepwise removal of 5' mononucleotides to yield blunt end fragments at optimally 37°C, 30 mM sodium acetate pH 4.5, 50 mM NaCl, 1 mM $ZnCl_2$ and 0.5% glycerol |
| Bal 31 nuclease | Boehringer Mannheim | Cleaves 3' ends of double stranded nucleic acid by hydrolysis of phosphodiester bonds and stepwise removal of 5' mono-nucleotides from 3' hydroxyl termini of double stranded nucleic acid (optimal temperature is 37°C in 66 mM TRIS-HCl pH 8.0 and 6 mM $MgCl_2$ |
| S1 nuclease | Boehringer Mannheim | Cleaves 3' ends of single stranded nucleic acid by complete degradation optimally at 37°C in 200 mM NaCl, 50 mM sodium acetate pH 4.5, 1 mM Zn $SO_4$ and 0.5% glycerol |

The above table is not intended as a listing of all enzymes which cleave nucleic acid in an endonuclitic or exonuclitic manner, but rather, an exemplary listing. Those skilled in the art will be able to identify similar enzymes based on vendor and supplier catalogs, publications and general knowledge in the art.

Preferably, the biological sample is exposed to the enzyme combination in a suitable incubation buffer. A preferred buffer consists of: 50 mM sodium acetate, pH4.5; 100 mM NaCl; 1 mM $ZnSO_4$; 1.5 mM $MgCl_2$; and 1% glycerol. Incubation times and temperatures are primarily dependent upon the type of biological sample and the type of microorganism, and can be determined by those skilled in the art without undue experimentation. It has been found that for a solubilized human sputum sample containing Mycobacterium tuberculosis, incubation of the sample and enzyme combination at about 37°C for at least 15 minutes is adequate for removal of sufficient non-target nucleic acid to permit detection of the DNA of M. tuberculosis from as few as 10 organisms in a sample containing 100 micrograms of human DNA following amplification.

The biological samples which are exposed to the enzyme combination are any samples which may contain a microorganism, and thus include blood, plasma, sputum, urine, semen, saliva, pleural fluids, bronchial aspirates, nasopharyngeal samples, cervical mucous, cerebrospinal fluid, peritoneal fluid, feces, and tissue. As is recognized by those skilled in the art, certain biological samples are more likely to contain certain microorganisms, and thus different biological samples are preferred for detection of the nucleic acid of different microorganisms such as bacteria, viruses, mycobacteria, mycoplasmas, and fungi. For example, M. tuberculosis is most commonly found in sputum samples, streptococcal, staphylococcal and other bacterial organisms are commonly found in nasopharyngeal samples and bronchial aspirates, and viral organisms such as HIV are commonly found in serum and plasma.

As is also recognized by those skilled in the art, certain biological samples may require certain manipulation such as liquification, dilution, and homogenation, prior to exposure to the enzyme combination in order to optimize detection of the microorganism nucleic acid. Similarly, after exposure of the biological sample to the enzyme combination, the cleaved non-target nucleic acid is preferably removed from the sample by washing, centrifugation, affinity capture and/or hybridization, prior to lysis of the cells of the microorganisms. As with other manipulations of the sample, those skilled in the art are aware of appropriate lysis techniques which include heat, chemical, enzymatic, and physical/mechanical methods.

Lysis of the cells releases the microorganism's nucleic acid to be detected. This nucleic acid may be DNA or RNA depending on the particular microorganism. Similarly, the non-target nucleic acid may be DNA or RNA and may be double stranded and/or single stranded.

Generally, the amount of microorganism nucleic acid released is insufficient for reliable detection, and therefore is subjected to an appropriate amplification process. Suitable amplification procedures which are well known to those skilled in the art include: polymerase chain reaction (PCR) as taught in U.S. Patents 4,683,195 and 4,683,202; Strand Displacement Amplification (SDA) as taught by Walker et al., Nuc. Acids Res. 20, 1691 (1992); ligase chain reaction (LCR) as taught by Barany (1991) Proc. Nat. Acad Sci. U.S.A.88 189-193; 3SR amplification as taught by Guatelli et al. (1990) Proc. Natl. Acad. Sci. U.S.A. 87 1874-8; and Q-Beta amplification as taught by Lizardi et al. (1988) Biotech 6 1197-1202.

The invention is further described by the following examples which are offered by way of illustration and are not intended to limit the invention in any manner. In these examples all percentages are by weight if for solids and by volume if for liquids, and all temperatures are in degrees Celsius unless otherwise noted.

## EXAMPLE 1

Pre-treatment of Biological Sample with DNase I to Degrade Non-target DNA

### Purpose

This experiment was an attempt to improve upon Barker's published lysis method for PCR (Sritharan et al. Molecular & Cellular Probes vol. 5 (1991) pp. 385-6) by using DNase I to remove non-target DNA prior to cell lysis.

### Method

Frozen human sputum samples (smear AFB negative) were thawed to room temperature. Two (2) mls of BACTEC®-cultured M. tuberculosis organisms (~8x10⁶) were pelleted and added to the samples. Other samples were maintained as negative controls.

The samples were then liquified with NALC (1% N-acetyl-L-cysteine in 100mM sodium citrate and 0.5 N sodium hydroxide) and incubated at room temperature for 15 minutes, then brought up to 50 mls total volume with 50mM potassium phosphate (pH 7.5), before being spun in a clinical centrifuge at 3000 x g for 20 minutes. The supernatants were decanted, and the sediments were reconstituted into 2ml of water in microcentrifuge tubes, and were spun at 12,000 x g for 5 minutes to pellet, and again were decanted.

One half of the samples were reconstituted into 500 μl TE buffer (10mM Tris-HCl 1mM EDTA pH 8.0) with 1% Triton X-100, then incubated at 100° C for 15 minutes. The other half were incubated in 500 μl DNase buffer (0.1M sodium acetate and 5mM magnesium sulfate pH 5.0) and 100 Units of DNase I for 15 minutes at 37°C, after which Triton X-100 was added to a final concentration of 1% and incubated the samples at 100°C for 15 additional minutes.

Both sets of samples were serially diluted in sterilized tubes up to 1/10,000 fold. Then 5ul aliquots were added to standard PCR mixtures and amplified using M. tuberculosis- complex specific primers cycled at 94°C for 1 minute, 62°C for 1 minute, and 72°C for 1 minute for 30 cycles (M. tuberculosis complex consists of M. tuberculosis, M. bovis, M. bovis-BCG, M. africanum and M. microti.). Portions of each PCR mixture were electrophoresed on 10% acrylamide gels.

### Results

The samples that were simply boiled yielded similar results to the DNase I-treated samples. DNase I cut non-target DNA into pieces less than 1000 base pairs in size. By either method, sensitivity is less than 10 M. tuberculosis organisms indicating that the PCR was not compromised by DNase I treatment.

## EXAMPLE 2

Treatment of Human DNA with a 3-Enzyme Combination.

Method

A 50 µl aliquot of enzyme buffer (100mM sodium citrate pH 5.0, 5mM magnesium sulfate, 1mM calcium phosphate, 1mM zinc sulfate) was added to 0.6ml sterile tubes. To each tube was also added 2.5 µg human placental DNA (Sigma).

The following enzymes were then used in the following runs on the tubes:

Enzymes:

DNAse I 100 units=10 µl (10,000 U/ml)
Exonuclease III 100 Units=1 µl (100,000 U/ml)
Mung Bean Nuclease 10 Units=1 µl (10,000 U/ml)

Samples:

1. Untreated; incubated at 37° C for 30 minutes
2. Untreated, incubated at 37° C for 30 minutes and then at 100° C for 5 minutes
3. 100 Units of DNase I, incubated at 37° C for 30 minutes and then at 100° C for 5 minutes
4. 100/10 Units of Exonuclease III/Mung Bean Nuclease, incubated at 37° C for 30 minutes and then at 100° C for 5 minutes.
5. 100/100/10 Units of DNase I/Exonuclease III/Mung Bean Nuclease, incubated at 37° C for 30 minutes and then at 100° C for 5 minutes.
6. Incubated at 100° C for 5 minutes, then cooled to 4° C; then 100/100/10 units of DNase I/Exonuclease III/Mung Bean Nuclease, incubated at 37° C for 30 minutes and then at 100° C for 5 minutes.
7. 100/100/10 units of DNase I/Exonuclease III/Mung Bean Nuclease, incubated at 37° C for 30 minutes.

Following the runs, all samples were phenol/chloroform extracted and ethanol precipitated for 2 days at -20° C as described below.

Phenol Chloroform Procedure:

Add equal volumes of sample to be extracted and a 50:48:2 solution of buffered phenol, chloroform, isoamyl alcohol. Vortex mix, then centrifuge the sample at 12000 x g for 1 minute. The DNA is present in the upper phase, while proteins are trapped at the interface and the lower phase. Follow with ethanol precipitation of the upper phase by adding 2 volumes 70 % ice cold ethanol and 1/10th volume 3M sodium acetate pH 4.5. Then samples were subjected to microcentrifugation at 12,000 x g and removal of the supernatant.

The samples were dried, and reconstituted into 17 µl sterile water and 3 µl gel loading dye. (25% Ficoll-400, 0.25% xylene cyanol and 0.25% bromophenol blue dyes) and electrophoresed onto 1% agarose gels.

Results

DNase I completely degraded 2.5 µg of human DNA. Also, a combination of Exonuclease III and Mung Bean Nuclease significantly reduced the size fragments of the DNA. The use of all 3 enzymes together completely eliminated both single stranded and double stranded DNA as shown in Figure 1 which is a photograph of a 1% agarose gel electrophoresed in 1 x TAE buffer where the lane assignments are as follows:

Lane

1 23 kb DNA ladder
2 untreated DNA (Sample 1)
3 boiled DNA (Sample 2)
4 DNase I - treated DNA (Sample 3)
5 Exonuclease III/Mung Bean Nuclease-treated DNA (Sample 4)
6 DNase I/Exonuclease III/Mung Bean Nuclease-treated DNA (Sample 5)
7 pre-boiled DNA subsequently treated with DNase I/Exonuclease III/Mung Bean Nuclease (Sample 6)

8 DNase I/Exonuclease III/Mung Bean Nuclease-treated DNA not pre-boiled (Sample 7)

**EXAMPLE 3**

Ability of 3-Enzyme Combination to Eliminate Both Single- and Double-stranded DNA and Evaluation of S1 Nuclease on Sample DNA

Purpose

This experiment tested the ability of S1 Nuclease, as well as the three enzymes of Example 2, to cut and eliminate both single- and double-stranded DNA.

Method

Fourteen (14) tubes were set up, each one receiving 5 μg of human genomic DNA in a 10 μl volume. Each sample received 10 μl of a 10x concentration buffer that was appropriate for each enzyme, (see below for composition), and all were brought up to 100 μl with sterile water. Prior to enzyme incubation, tubes 8-14 were incubated at 100° C for 5 minutes, then quick-cooled to 20° C.

Samples:

| | |
|---|---|
| 1. Untreated DNA | 8. Boiled DNA |
| 2. + DNase I | 9. + DNase I |
| 3. + Exonuclease III | 10. + Exonuclease III |
| 4. + Mung Bean Nuclease | 11. + Mung Bean Nuclease |
| 5. + S1 Nuclease | 12. + S1 Nuclease |
| 6. + DNase I/S1 | 13. + DNase I/S1 |
| 7. + DNase I/Mung Bean/Exo III | 14. + DNase I/Mung Bean/Exo III |

The following amounts of enzymes were added to the designated runs above:
DNase I= 25 Units = 2.5 μl of 10,000U/ml stock
Exo III= 100 Units = 1 μl of 100,000 U/ml stock
Mung Bean Nuclease=10 Units= 1 μl of a 10,000 U/ml stock
S1 Nuclease= 10 Units = 1/100 dilution of a 400U/ml stock
The following buffers were used in the runs containing the corresponding enzymes:

10x S1 Nuclease Buffer

2 M Sodium Chloride
0.5 M Sodium Acetate pH 4.5
10mM Zinc Sulfate
5% Glycerol

10x Exonuclease III Buffer

0.66 M Tris-Hydrochloride pH 8.0
66 mM Magnesium Chloride

10x Mung Bean Nuclease Buffer

300 mM Sodium Acetate pH 4.5
500 mM Sodium Chloride
10 mM Zinc Chloride

7

50% Glycerol

10x DNase I Buffer

1 M Sodium Acetate pH 4.5
10mM Magnesium Sulfate
10mM Calcium Phosphate

All samples were incubated at 37° C for 30 minutes, and then at 100° C for 5 minutes. Then, all samples were phenol/chloroform extracted and ethanol precipitated as in Example 2. The samples were reconstituted into 20 μl sterile water plus 3 μl loading dye, and electrophoresed on a 1% agarose gel, followed by ethidium bromide staining.

Results

The enzymes degraded the DNA to varied amounts. For double-stranded DNA (Samples 1-7), the DNase I was the most effective enzyme. For the single-stranded DNA (Samples 8-14), the Exo III by itself was not very effective, but the S1 nuclease worked very well. Also, a combination of DNase I and Mung Bean Nuclease worked very well. The best results were obtained by the 3-enzyme combination, which showed total degradation with either the double or single stranded DNA source. These results suggested the combination of DNase I, which is very effective with double-stranded DNA, and Mung Bean Nuclease and S1 Nuclease, which are very effective with single-stranded DNA.

**EXAMPLE 4**

Treatment of Biological Sample with Combination of Enzymes Prior to Lysis of Mycobacterium tuberculosis Cells

Method

M. tuberculosis organisms were prepared by harvesting a 1 ml aliquot of cells in culture at $10^6$ organisms/ml by centrifugation at 12000 x g for 5 minutes and removal of the supernatant. The pellet was then serially diluted to $10^4$ organisms per ml in sterile water. A 10 μl aliquot of the diluted M. tuberculosis was added to each of 10 tubes and human DNA from two stocks (0.5 μg/μl and 2.5 μg/μl) was also added to each tube as follows:

| Tube # | Human DNA Amount(μg) |
|--------|----------------------|
| 1      | 0                    |
| 2      | 1                    |
| 3      | 5                    |
| 4      | 10                   |
| 5      | 25                   |
| 6      | 0                    |
| 7      | 1                    |
| 8      | 5                    |
| 9      | 10                   |
| 10     | 25                   |

The following combination of the enzymes was added to tubes 6-10:
DNase I (25 Units)
S 1 Nuclease (10 Units)
Mung Bean Nuclease (10 Units)

Each tube was brought up to 100 μl in 1x buffer (50mM sodium acetate pH 4.5, 100 mM sodium chloride, 1 mM zinc sulfate, 1% glycerol) after enzyme addition. The tubes were incubated at 37° C for 30 minutes, after which, Triton X-100 was added to each tube to a final concentration of 1%. All tubes were then incubated at 100° C for 5 minutes.

A 5 μl aliquot of each sample containing only 5 organisms was added to standard PCR mixtures that contained 50 μl volume of 50 mM TRIS pH 8.8, 0.5 mM dNTP's, 4.5 mM MgCl$_2$, 10 mM β-mercaptoethanol, 2.5 units Taq polymerase, and 0.25 uM primers coding for a 74 base pair fragment of insertion sequence 6110 of M. tuberculosis. Positive and negative controls were also run under the following parameters:

| Temp (°C) | Time | Activity |
|---|---|---|
| 94 | 3 min | Denaturation |
| 94 | 1 min | Denaturation |
| 62 | 1 min | Annealing |
| 72 | 1 min | Extension |
| (Above 3 steps cycled 30 times) | | |
| 72 | 7 min | Extension |
| 4 | Overnight | Storage |

Following PCR, 10% of each product was removed and electrophoresed on 10% acrylamide gels, followed by ethidium bromide visualization. The remainder of the samples were phenol/chloroform extracted and ethanol precipitated as in Example 2, reconstituted into 20 μl water and 3 μl dye, and electrophoresed onto a 1 % agarose gel, followed by ethidium bromide visualization.

Results

The photographs of the gels in Figures 2, 3 and 4 show that PCR amplifies product in up to 25 μg of initial human DNA, both with and without the enzyme treatments. More amplified product is seen in the samples that did not receive enzyme treatments, but less non-specific product (i.e. non-target nucleic acid) is seen in the samples that did receive enzyme treatment. The agarose gel showed that the enzyme combination of this Example degraded up to 25 μg of human DNA completely in 30 minutes thereby permitting detection of only target (i.e. M. tuberculosis nucleic acid).

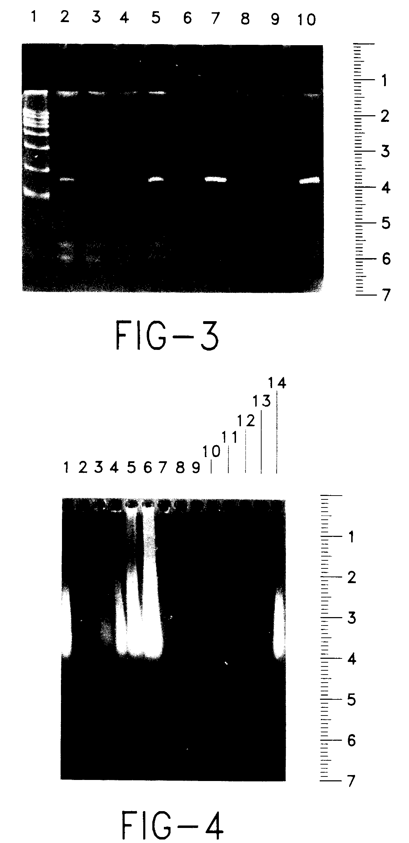

Specifically, Figure 2 shows untreated samples run on a 10% acrylamide gel in 1 x TBE. The gel shows good PCR sensitivity, but non-specific bands. Figure 3 shows the treated samples also run on a 10% acrylamide gel in 1 x TBE. This gel shows less sensitivity than that in Figure 2, but there are no non-specific bands detected. Figure 4 shows a 1% agarose gel in which the right half of the gel shows total degradation of up to 25 μg of human DNA by the enzyme combination. The lane assignments for Figures 2 and 3 are the same and are as follows:

Lane

1 biomarker standards
2 0 μg human DNA
3 1 μg human DNA
4 5 μg human DNA
5 10 μg human DNA
6 25 μg human DNA
7 10$^3$ copies of linearized SK4.3 plasmid containing the M. tuberculosis complex DNA region
8 PCR mix alone
9 H$_2$O negative
10 10$^3$ copies of linearized SK4.3 plasmid in buffer (universal buffer in Figure 2 and TE buffer in Figure 3).

The lane assignments for Figure 4 are as follows:

Lane

1 23 kb ladder
2 0 µg human DNA )
3 1 µg human DNA )
4 5 µg human DNA ) untreated
5 10 µg human DNA)
6 25 µg human DNA)
7 blank
8 blank
9 0 µg human DNA )
10 1 µg human DNA )
11 5 µg human DNA ) treated
12 10 µg human DNA)
13 25 µg human DNA)
14 23 kb ladder

## EXAMPLE 5

Treatment of Non-target DNA with a Combination of Enzymes Prior to Lysis of M. tuberculosis Cells

Method

The following amounts of human DNA were made up in 100 µl total volume in duplicate (all samples were in the following "universal" buffer: 50 mM sodium acetate pH 4.5, 100 mM sodium chloride, 1 mM zinc sulfate, 1.5 mM magnesium chloride, 1% glycerol.).

Tubes:

1. 0 µg DNA
2. 10 µg DNA
3. 25 µg DNA
4. 50 µg DNA
5. 100 µg DNA

All tubes also contained 100 M. tuberculosis organisms.

One set of tubes was incubated at 37° C for 15 minutes, the other set was incubated with the following enzyme combination for 15 minutes at 37° C:

| | |
|---|---|
| DNase I | 50 Units |
| Mung Bean Nuclease | 25 Units |
| S1 Nuclease | 25 Units |

Five (5) other tubes, each one containing 100 µg human DNA and the same universal buffer were incubated with the following enzymes for 15 minutes at 37° C:

1. 50 Units DNase I
2. 25 Units Mung Bean Nuclease
3. 25 Units S1 Nuclease
4. All 3 of the above enzymes at the above concentrations
5. No enzyme treatment.

After 15 minutes, all tubes were incubated at 100° C for 5 minutes. From the first two sets of samples, 10 µl of each sample was placed into PCR mixes, and amplified as in Example 4. The remainder of each sample (all three sets) was phenol/chloroform extracted and ethanol precipitated as in Example 2, followed by reconstitution and electrophoresis onto 1% agarose gels. Then, 10% of each PCR-amplified product was electrophoresed onto 10% acrylamide gels, followed by ethidium bromide visualization.

Results

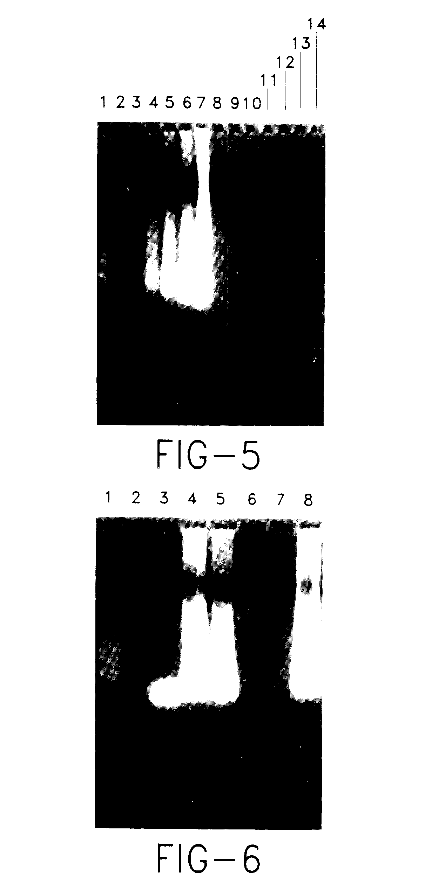

When no enzyme treatment was performed, PCR did not detect the 100 M. tuberculosis organisms in the presence of 100 µg of non-target DNA. With enzyme treatment, no amplification was seen, but the agarose gel showed that the 3-enzyme mixture was capable of degrading 100 µg of non-target DNA in 15 minutes. All three enzymes were necessary to completely degrade the 100 μg of DNA. Specifically, the gel in Figure 5 shows that the 3-enzyme treatment degrades up to 100 μg of human DNA, and Figure 6 shows that all three enzymes were necessary to achieve total degradation. After this experiment, it was proposed to include a washing step after the enzyme treatment step as one possible alternative to prevent target DNA degradation by the enzymes. The lane assignments in Figure 5 are as follows:

Lane

1 23 kb ladder
2 blank
3 0 μg human DNA )
4 10 μg human DNA )
5 25 μg human DNA ) untreated
6 50 μg human DNA )
7 100 μg human DNA)
8 blank
9 blank
10 0 μg human DNA )
11 10 μg human DNA )
12 25 μg human DNA ) treated
13 50 μg human DNA )
14 100 μg human DNA)

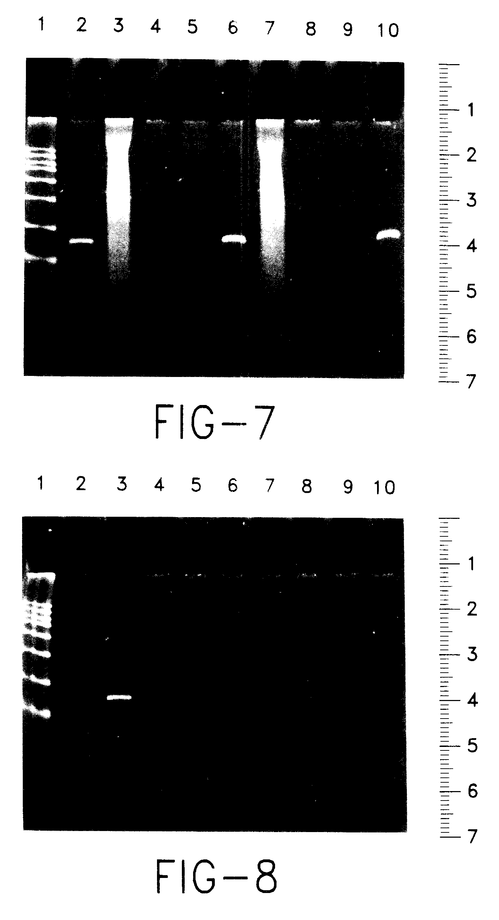

The lane assignments for Figure 7 are as follows:

Lane

1 23 kb ladder
2 blank
3 100 μg human DNA - DNase I only
4 100 μg human DNA - Mung Bean Nuclease only
5 100 μg human DNA - S1 Nuclease only
6 100 μg human DNA - DNase I/Mung Bean Nuclease/S1 Nuclease
7 blank
8 100 μg human DNA - untreated

## EXAMPLE 6

Removal of Non-target DNA by Enzymatic Means, Followed by a Wash Step, Prior to Lysis of Mycobacterium tuberculosis Organisms

Method

Eight (8) tubes containing 100 μg of non-target human DNA in 1x universal buffer (see Example 5) (100 μl total volume)were prepared. Tubes 1-4 each received 100 M. tuberculosis organisms. Four (4) types of treatments were performed on the samples as follows:

1. (Tubes 1 and 5) No enzyme treatment. Sample incubated at 37° C for 30 minutes, Triton-X-100 was added to a final concentration of 1% and the sample was incubated at 100° C for 5 minutes, followed by a 15 second microcentrifugation step at 12000 x g.

2. (Tubes 2 and 6) Treated with 50 Units DNase I, 25 Units Mung Bean Nuclease, and 25 Units of S1 Nuclease for 30 minutes at 37° C. This was followed by addition of Triton-X-100 to 1% final concentration incubation at 100° C for 5 minutes, and a 15 second microcentrifugation step at 12,000 x g.

3. (Tubes 3 and 7) Treated with the same enzymes as in treatment #2, then Triton X-100 was added to 1% final concentration, and EDTA was added to 1 mM final concentration. This was followed by incubation at 100° C for 5 minutes, and a 15 second microcentrifugation step at 12,000 x g.

4. (Tubes 4 and 8) Treated with the same enzymes as in treatment #2, followed by a 5 minute microcentrifugation at 12,000 x g. Supernatants were pipetted off, and the pellets were resuspended into 100 μl TE buffer + 1% Triton-X-100, followed by incubation at 100° C for 5 minutes.

Seven (7) additional samples, each one containing 100 μg human non-target DNA, were combined with

$10^6$, $10^5$, $10^4$, $10^3$, $10^2$, $10^1$ and $10^0$ M. tuberculosis organisms. These samples were treated as in treatment #2.

A 25 µl aliquot of each sample (i.e. 25 M. tuberculosis organisms in tubes 1-4) was amplified by PCR as in Example 4, with 25 µl of sterile water also being added to each mix. The samples that did not receive any M. tuberculosis organisms previously (tubes 5-8) received 1000 copies of a plasmid containing an M. tuberculosis target sequence. Ten percent (10%) of each PCR product was electrophoresed onto 10% acrylamide gels, followed by ethidium bromide visualization.

Results

Results indicated that the incorporation of a wash step (treatment #4) enhanced the detection of the M. tuberculosis nucleic acid. No amplification is seen when the sample is not subjected to enzymatic treatment (#1), but some amplification is seen when only enzymatic treatment with subsequent heat lysis is performed (#2 and #3). However, the best results were seen with enzyme treatment, followed by subsequent washing to remove the enzymes prior to heat lysis (#4). Specifically, Figure 7 shows that both the M. tuberculosis genomic DNA at 100 copies per reaction and plasmid containing the M. tuberculosis DNA region was recognized by the PCR primers as evidenced by the bands in lanes 6 and 10. The lanes in Figure 7 represent the following:

Lane

1 DNA standards
2 positive control-$10^2$ organisms
3 treatment #1 with $10^2$ organisms
4 treatment #2 with $10^2$ organisms
5 treatment #3 with $10^2$ organisms
6 treatment #4 with $10^2$ organisms
7 treatment #1 plus plasmid
8 treatment #2 plus plasmid
9 treatment #3 plus plasmid
10 treatment #4 plus plasmid

Reviewing the organism titration (i.e. the additional 7 tubes), the only samples that were detected were the positive amplification control, and the $10^6$ sample. This was probably due to the use of too much enzyme, and the lack of a washing step. Figure 8 shows the positive control in lane 3 and the $10^6$ sample in lane 10. The lanes in Figure 8 represent the following:

Lane

1 DNA standards
2 blank
3 positive control with $10^2$ organisms
4 $10^0$ organisms
5 $10^1$ organisms
6 $10^2$ organisms
7 $10^3$ organisms
8 $10^4$ organisms
9 $10^5$ organisms
10 $10^6$ organisms

**EXAMPLE 7**

Optimization of the Treatment of a Biological Sample with a Combination of Enzymes and a Washing Step Prior to M. tuberculosis Lysis and Amplification

Method

From three (3) stocks, the following samples were created, each one in 1x universal buffer (as in Example 5). All samples were 100 µl total volume.

1. $10^6$ M.tb. organisms + 100 µg human non-target DNA.

2. $10^5$ M.tb. organisms + 100 μg human non-target DNA.

3. $10^4$ M.tb. organisms + 100 μg human non-target DNA.

4. $10^3$ M.tb. organisms + 100 μg human non-target DNA.

5. $10^2$ M.tb. organisms + 100 μg human non-target DNA.

6. $10^1$ M.tb. organisms + 100 μg human non-target DNA.

7. $10^0$ M.tb. organisms + 100 μg human non-target DNA.

8. 0 M.tb. organisms + 100 μg human non-target DNA.

To each of the above samples, the following combination of enzymes was added:

DNase I: 50 Units

Mung Bean Nuclease: 25 Units

S1 Nuclease: 25 Units

The samples were incubated at 37° C for 15 minutes and then microcentrifuged at 12000 x g for 5 minutes. The supernatants were pipetted off, and discarded. Each pellet was then brought up into 100 μl TE buffer + 1% Triton X-100 and incubated for 15 minutes at 100° C. Samples were allowed to cool and then 25 μl of each sample was amplified by PCR as in Example 4, and detected as in Example 4.

## Results

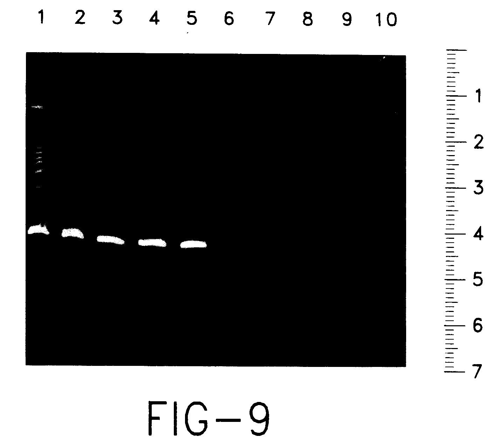

Amplification was observed from $10^6$ input organisms, all the way down to 101 organisms. The $10^0$ sample was indeterminate, and the positive and negative amplification controls were as expected. No residual non-target DNA was observed on the gel. Specifically, Figure 9 shows a 10% acrylamide gel on which an amplified sample of 10 M. tuberculosis organisms in 100 μg of initial human DNA was visualized after digestion of the DNA. The lanes in Figure 9 represent the following:

## Lane

1 DNA standards and positive control

2 $10^6$ organisms

3 $10^5$ organisms

4 $10^4$ organisms

5 $10^3$ organisms

6 $10^2$ organisms

7 $10^1$ organisms

8 $10^0$ organisms

9 negative control (human DNA)

10 $H_2O$ control

This Example shows that the technique of the present invention allows detection of the DNA from fewer than 10 M. tuberculosis organisms in the presence of 100 μg of initial human non-target DNA.

Other modifications of the above-described embodiments of the invention which are obvious to those of skill in the art of molecular biology and related disciplines are intended to be within the scope of the following claims.

## Claims

1. A method for removal of contaminating nucleic acid from a biological sample which may contain a microorganism with nucleic acid to be detected, said method comprising exposing the biological sample to a combination of enzymes, said combination comprising an enzyme which cleaves nucleic acid in an endonuclitic manner and an enzyme which cleaves nucleic acid in an exonuclitic manner.

2. The method of claim 1 wherein the combination of enzymes comprises:
   (a) an enzyme which cleaves double stranded nucleic acid;
   (b) an enzyme which cleaves 3' ends of nucleic acid; and
   (c) an enzyme which cleaves 5' ends of single stranded nucleic acid.

3. The method of claim 2 wherein the enzyme which cleaves double stranded nucleic acid is DNase I.

4. The method of claim 2 wherein the enzyme which cleaves 3' ends of nucleic acid cleaves 3' ends of single stranded nucleic acid.

5. The method of claim 4 wherein the enzyme which cleaves 3' ends of single stranded nucleic acid is S1 nuclease.

6. The method of claim 2 wherein the enzyme which cleaves 3' ends of nucleic acid cleaves 3' ends of double stranded nucleic acid to yield single stranded nucleic acid overhangs.

7. The method of claim 6 wherein the enzyme which cleaves 3' ends of double stranded nucleic acid is Exonuclease III.

8. The method of claim 2 wherein the enzyme which cleaves 5' ends of single stranded nucleic acid is Mung Bean Nuclease.

9. The method of claim 2 wherein the contaminating nucleic acid is DNA.

10. The method of claim 9 wherein the contaminating DNA is double stranded.

FIG-1

FIG-2

FIG-3

FIG-4

FIG-5

FIG-6

FIG-7

FIG-8

FIG-9

## EUROPEAN SEARCH REPORT

European Patent Office

Application Number

EP 94 30 9336

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | WO-A-86 06743 (A/S ALFRED BENZON) 20 November 1986<br>* page 32, line 1 - line 2 *<br>* page 33, line 19 - page 34, line 3 *<br>--- | 1-4 | C12N1/08<br>C12N1/06<br>C12Q1/68 |
| X | MOLECULAR CLONING, VOL.1, A LABORATORY MANUAL, 1989, CSH, NY,US;<br>pages 2.73 - 2.76<br>SAMBROOK, FRITSCH, MANIATIS<br>* page 2.73, line 6 - line 13 *<br>--- | 1-4 | |
| A | EP-A-0 430 270 (BEHRINGWERKE) 1 December 1989<br>* the whole document *<br>--- | 1-10 | |
| A | EP-A-0 547 789 (BECTON DICKINSON AND COMPANY) 23 June 1993<br>* the whole document *<br>--- | 1-10 | |
| P,A | EP-A-0 626 456 (BECTON, DICKINSON AND COMPANY) 30 November 1994<br>* the whole document *<br>----- | 1-10 | TECHNICAL FIELDS SEARCHED (Int.Cl.6)<br><br>C12N<br>C12Q |

The present search report has been drawn up for all claims

| Place of search | Date of completion of the search | Examiner |
|---|---|---|
| THE HAGUE | 14 March 1995 | Hornig, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)